# FASCICULE DE BREVET EUROPEEN

(11) **EP 1 017 361 B1**
(45) Date de publication et mention de la délivrance du brevet: **10.04.2002**
(21) Numéro de dépôt: 98942819.8
(22) Date de dépôt: 03.09.1998
(51) Int. Cl.: A61K 7/16

(54) **FORMULATION BUCCODENTAIRE COMPRENANT DES NANOFIBRILLES DE CELLULOSE ESSENTIELLEMENT AMORPHES**
Mundpflegemittel enthaltend wesentlich amorphen Cellulose-Nanofibrillen
BUCCODENTAL FORMULATION COMPRISING SUBSTANTIALLY AMORPHOUS CELLULOSE NANOFIBRILS

(30) Priorité: 22.09.1997 FR 9711767
(43) Date de publication de la demande: 12.07.2000
(73) Titulaire: RHODIA CHIMIE, 92408 Courbevoie Cédex (FR)
(72) Inventeur: CANTIANI, Robert, F-92800 Puteaux (FR); WILLEMIN, Claudie, F-75008 Paris (FR)
(74) Mandataire: Dubruc, Philippe
(86) Numéro de dépôt international: FR9801888
(87) Numéro de publication internationale: WO9915141

(56) Documents cités:
- EP-A- 0 198 094
- EP-A- 0 726 356
- WO-A-98/02486
- WO-A-98/02487

## Description

La présente invention se rapporte à la mise en oeuvre de nanofibrilles de cellulose essentiellement amorphes dans des formulations buccodentaires et aux formulations ainsi obtenues.

La présente invention a plus précisément pour objet de proposer un agent épaississant dans des formulations buccodentaires présentant une meilleure stabilité thermique et ionique que les agents épaississants classiques, sans préjudice des autres avantages requis.

Usuellement, les formulations buccodentaires incorporent des agents humectants, de type glycérol, polyéthylèneglycol, sorbitol, des abrasifs minéraux comme la silice amorphe, des phosphates dicalciques, hydratés ou non, du carbonate de calcium, des agents tensioactifs lavants, des agents actifs de type antibactériens comme la chlorhexidine, les dérivés du fluor, les enzymes et les édulcorants comme le saccharinate de sodium ainsi que de nombreux autres composants contribuant plus particulièrement à conférer auxdites formulations un aspect, un goût particulier et/ou des propriétés spécifiques.

Parmi l'ensemble de ces composés, certains se présentent sous une forme hydrosoluble alors que d'autres en revanche, ont un aspect poudre solide. Pour les formuler en un mélange homogène, il est donc nécessaire d'incorporer, en outre dans la formulation, au moins un agent annexe ayant plus particulièrement un rôle de liant à l'égard de ces différents composés. Parallèlement à cette fonction, cet agent contribue généralement à conférer à la formulation buccodentaire, un comportement rhéologique adéquat à l'application envisagée et en particulier vise à l'épaissir. A titre représentatif de ces épaississants conventionnels des formulations buccodentaires, on peut mentionner les silices épaississantes, les alginates, les polysaccharides de type gommes naturelles comme les carraghénanes, la xanthane, et le guar et tout particulièrement les dérivés de cellulose comme la carboxméthylcellulose et l'hydroxyéthylcellulose.

La demande de brevet EP-A-0 198 094 divulgue une formulation buccodentaire comprenant un agent liant constitué d'une cellulose microfibrillée à parois secondaires additivée avec de la carboxyméthyl cellulose. La quantité de carboxyméthyl cellulose employée varie entre 30% et 600% du contenu solide en cellulose. L'agent épaississant est employé dans la formulation en quantités de l'ordre de 1 à 1.5% en poids.

En fait, les épaississants de type dérivés de cellulose actuellement disponibles dans des formulations buccodentaires ne sont pas totalement satisfaisants.

Il est nécessaire d'en employer des quantités importantes, de l'ordre de 1 % environ, pour obtenir une viscosité convenable. Par ailleurs, les formulations les incorporant sont sujettes à des variations importantes de viscosité lorsqu'elles sont confrontées à des températures élevées ou comprennent une charge ionique importante.

Enfin, les formulations à base de ces dérivés de cellulose 'ne donnent pas totalement satisfaction sur le plan esthétique. C'est ainsi que les compositions de type pâte dentaire, épaissies par ces dérivés de cellulose, ne possèdent pas une bonne tenue sur la brosse à dent.

La présente invention a précisément pour objet de proposer un agent épaississant dans des formulations buccodentaires, dénué des inconvénients précités et doté en revanche d'un comportement avantageux en termes rhéologiques.

La demanderesse a ainsi mis en évidence que les nanofibrilles de cellulose, essentiellement amorphes et avantageusement issues de parois primaires, satisfaisaient avantageusement à ces deux impératifs. De manière inattendue, ce type de fibres de cellulose manifeste une bonne compatibilité à l'égard des autres composants présents dans les compositions buccodentaires.

D'une manière générale, la cellulose native se présente toujours sous une forme fibrillaire. Ses fibrilles sont des matériaux bien connus qui sont en particulier déjà proposés pour modifier la texture des milieux dans lesquels elles sont introduites. Dans le cas des milieux fluides, ils modifient leurs viscosités voire leurs profils rhéologiques.

Les fibrilles de cellulose peuvent être d'origine diverse, par exemple d'origine végétale, bactérienne, animale, fongique ou amibienne.

Les fibrilles de cellulose sont habituellement fortement associées entre elles, dans les parois ou les fibres. On distingue les parois secondaires, se trouvant pour l'essentiel dans le bois, des parois primaires dont un exemple typique est la parenchyme. Des exemples de parenchyme sont constitués par la pulpe de betterave sucrière, les citrus (citrons, oranges, pamplemousses) et la plupart des fruits et légumes.

Dans les parois secondaires, ces fibrilles sont organisées sous forme de nappes très orientées formant ainsi une fibre indissociable. Elles se présentent classiquement sous la forme d'agrégats de quelques dizaines de nanomètres à quelques micromètres. Ces agrégats sont constitués de microfibrilles élémentaires qui ne peuvent pas être désenchevêtrées, lors de leur homogénéisation sans provoquer leur cassure.

Dans le cadre de la présente invention, les fibrilles de cellulose considérées sont les nanofibrilles de cellulose, NFC, de préférence issues de cellules à parois primaires.

Par opposition aux microfibrilles de cellulose de parois secondaires discutées ci-dessus, elles présentent un diamètre d'au plus quelques nanomètres et ont l'aspect de filaments. Avantageusement, il est possible de démêler ces nanofibrilles de cellulose de parois primaires lors des étapes homogénéisation.

La présente invention a donc pour objet principal une formulation buccodentaire caractérisée en ce qu'elle comprend au moins un agent épaississant comprenant des nanofibrilles de cellulose essentiellement amorphes, possédant un taux de cristailinité inférieur ou égal à 50 %, lesdites nanofibrilles de cellulose étant présentes sous une forme associée à au moins un composé organique polyhydroxylé (polyOH) dans un rapport pondéral (polyOH) / [(polyOH) + (NFC)] inférieur ou égal à 30 %.

Dans la variante préférée de l'invention, les nanofibrilles de cellulose sont issues de cellules constituées de préférence d'au moins environ 80 % de parois primaires. De préférence, la quantité de parois primaires est d'au moins 85 % en poids.

Les nanofibrilles présenteront alors au moins 80 % de cellules à parois primaires.

Elles présentent de préférence une section comprise entre environ 2 et environ 10 nm. Plus préférentiellement, celle-ci est comprise entre environ 2 et environ 4 nm.

Dans le cadre de l'invention, les nanofibrilles considérées sont des nanofibrilles dites essentiellement amorphes par opposition aux fibrilles dites cristallines.

Par essentiellement amorphes, on entend définir des nanofibrilles dont le taux de cristallinité est inférieur ou égal à 50 %. Selon une variante particulière de la présente invention, ce taux de cristallinité est compris entre 15 % et 10 % et plus préférentiellement inférieur à 50 %.

Ces nanofibrilles de cellulose essentiellement amorphes sont particulièrement avantageuses au regard des microfibrilles cristallines en ce sens qu'elles sont dispersables en milieux aqueux, confèrent des propriétés rhéologiques bien spécifiques de type rhéofluidifiantes et sont stables que ce soit thermiquement ou en milieux à charges ioniques importantes. A cet égard, les nanofibrilles essentiellement amorphes selon l'invention, confèrent avantageusement une excellente viscosité aux formulations les incorporant et ceci pour une faible concentration, de l'ordre de 0,15 % en poids, contre 1 % pour les dérivés de celluloses classiques.

Cette bonne efficacité manifestée, à dose réduite, par les nanofibrilles de cellulose est en fait une conséquence de leur excellent comportement rhéologique en termes de viscosité et de pouvoir rhéofluidifiant.

Les nanofibrilles de celluloses, mises en oeuvre selon l'invention, manifestent par ailleurs une aptitude à renforcer les arômes présents dans les formulations buccodentaires dans lesquelles elles sont introduites. La perception sensorielle de l'arôme par l'utilisateur s'en trouve accentuée.

Selon un mode préféré de l'invention, les nanofibrilles de cellulose, entrant dans les formulations revendiquées sont chargées en surface en acides carboxyliques et en polysaccharides acides, seuls ou en mélange.

Par acides carboxyliques, on entend les acides carboxyliques simples, ainsi que leurs sels. Ces acides sont de préférence choisis parmi les acides uroniques et sont plus particulièrement l'acide galacturonique et/ou l'acide glucuronique.

En tant que polysaccharides acides, on peut citer les pectines dont plus particulièrement les acides polygalacturoniques. Ces polysaccharides acides peuvent être présentes en mélange avec des hémicelluloses.

En fait, ces nanofibrilles chargées en surface ne résultent pas d'un simple mélange entre lesdites nanofibrilles et les acides et polysaccharides. Il s'agit plutôt d'une combinaison étroite entre ces deux types de composés dérivant directement du procédé utilisé pour préparer les nanofibriiles. En effet, ce procédé de préparation peut être tel que les acides et polysaccharides ne sont pas totalement séparés des fibres mais demeurent en surface de ces dernières, leur conférant alors des propriétés bien spécifiques. Il est important de souligner que ces mêmes propriétés ne seront pas reproduites si l'on procède successivement à une séparation complète des nanofibrilles de ces acides et/ou polysaccharides puis à un rajout de ces derniers, aux nanofibrilles ainsi obtenues.

Les nanofibrilles de cellulose, obtenues à l'issue de procédés de ce type se présentent classiquement sous la forme de suspensions aqueuses dont la teneur en matières sèches est de l'ordre de 1 à 10 % en poids environ. Elles sont alors incorporées, telles quelles, ou sous une forme diluée, dans les formulations revendiquées.

De manière générale, les nanofibrilles sont présentes dans les formulations buccodentaires revendiquées à raison de 0,1 à 0,4 % et de référence de 0,15 à 0,3 % en poids.

Les nanofibrilles de celluose essentiellement amorphes y sont présentes sous une forme associée à un additif. Cet additif est en fait un agent permettant de formuler les nanofibrilles de cellulose sous une forme solide, redispersable.

A cet effet, l'additif est un composé organique polyhydroxylé (polyOH). Cette association, opérée lors du procédé de préparation des nanofibrilles de cellulose, a pour avantage de permettre la mise en forme desdites nanofibrilles, sous une forme sèche redispersable.

Le composé organique polyhydroxylé (pofyOH) est choisi de préférence parmi les carbohydrates et leurs dérivés et les polyalcools. A titre représentatif de ces carbohydrates, on peut tout particulièrement citer les monosaccharides linéaires ou cycliques en C₃ ou C₆ et de préférence en C₅ ou C₆ comme le fructose, le mannose, le galactose, le talose, le gulose, l'allose, l'altrose, l'idose, l'arabinose, le xylose, le lyxose et le ribose, les oligosaccharides comme le maltose et le lactose, les polysaccharides comme l'amidon, la cellulose, la gomme xanthane et le guar et leurs dérivés gras comme les sucroesters d'acides gras, les carbohydrates alcools de type sorbitol, mannitol, les carbohydrates acides comme les acides gluconique, uronique, galacturonique ainsi que leurs sels et les carbohydrates éthers comme les méthyl-, éthyl-, carboxyméthyl-, hydroxyéthyl- et hydroxypropyl-éthers de cellulose.

En ce qui concerne les polyalcools, il peut s'agir notamment du glycérol, du pentaérythrol, du propylène glycol, de l'éthylène glycol et/ou des alcools polyvinyliques.

Dans le cas particulier des carbohydrates et leurs dérivés, susceptibles d'être mis en oeuvre, conjointement avec les nanofibrilles de cellulose essentiellement amorphes, on peut plus particulièrement citer les celluloses carboxylées et de préférence la cellulose carboxyméthylée, désignée encore par CMC.

La cellulose est un polymère constitué d'unités monomériques de glucose. Le groupement carboxyté y est introduit de manière connue en soi, en faisant réagir l'acide chloroacétique avec la cellulose. Son degré de substitution correspond alors au nombre de groupement carboxyméthylés par unité de glucose. Le degré théorique maximal est de 3. Ces celluloses carboxylées sont dites de haut degré de substitution pour une valeur supérieure à 0,95 et de bas degré pour une valeur inférieure à celle-ci. En ce qui concerne ce degré de polymérisation, il varie dans de larges limites.

Ainsi, conviennent les celluloses carboxyméthylées de masses élevées, la teneur en cellulose carboxylée retenue étant alors inférieure ou égale à 30 % en poids, ou les celluloses carboxyméthylées de faibles masses, la teneur en cellulose carboxylée étant dans ce cas, plus particulièrement comprise entre 10 et 30 % en poids.

A masse identique, il s'avère par ailleurs possible d'en réduire encore la proportion par rapport aux nanofibrilles, en privilégiant le choix d'une cellulose carboxyméthylée à degré de substitution élevé.

De tels mélanges nanofibrilles de cellulose et cellulose carboxylé sont notamment décrits dans les demandes de brevet internationales WO 98/02486 et WO 98/02487.

De préférence, le composé organique polyhydroxylé (polyOH) est choisi parmi la carboxyméthylcellulose, la gomme xanthane, les guars, le sorbitol et leurs mélanges.

La formulation buccodentaire revendiquée comprend le ou les composés polyhydroxylés et les nanofibrilles de cellulose dans un rapport pondéral (polyOH)/[ (polyOH) + (NFC)] inférieur ou égal à 30 %.

Selon un mode privilégié de l'invention, la formulation buccodentaire comprend des nanofibrilles de cellulose sous une forme associée à de la carboxyméthylcellulose (CMC) de haut degré de substitution dans un rapport pondéral (CMC) / [(CMC) + (NFC)] inférieur ou égal à 20 %.

On peut également associer à la CMC un autre dérivé polyhydroxylé, comme le sorbitol par exemple. Dans ce cas particulier, le rapport pondéral (CMC) / [(potyOH) total + (NFC)] est alors abaissé significativement.

La composition solide redispersable de nanofibrilles de cellulose peut contenir outre le ou les composé(s) organique(s) polyhydroxylé(s) défini(s) ci-dessus, au moins un co-additif choisi parmi :
- les composés de formule (R1R2N)COA, dans laquelle R1 ou R2, identiques ou différents, représentent l'hydrogène ou un radical alkyle en C1-C10, de préférence en C1-C5, A représente l'hydrogène, un radical alkyle en C1-C10, de préférence en C1-C5, ou encore le groupement R'¹R'²N avec R'¹, R'², identiques ou différents, représentant l'hydrogène ou un radical alkyle en C₁-C₁₀, de préférence en C₁-C₅ et
- les tensioactifs de préférence anioniques, non ioniques ou amphotères, ces co-additifs pouvant être utilisés seuls ou en mélange.

Notons que l'utilisation de ces co-additifs permet, en combinaison avec l'additif comme par exemple la carboxyméthylcellulose, de renforcer le profil rhéofluidifiant des nanofibrilles de cellulose, après redispersion.

En ce qui concerne les composés du type (R¹R²N)COA, on préfère utiliser les composés comprenant deux fonctions amides. De préférence, on utilise l'urée comme co-additif.

A titre illustratif des tensioactifs amphotères, on peut citer sans intention de se limiter, les dérivés amphotères d'alkylpolyamines, les alkylbétaïnes, les alkyldiméthylbétaïnes, les alkylamidopropylbétaïnes, les alkylamidopropyldiméthylbétaïnes, les alkyltriméthylsulfobétaïnes, les dérivés d'imidazoline tels que les alkylamphoacétates, alkylamphodiacétates, alkylamphopropionates, alkylamphodipropionates, les alkylsultaïnes ou les alkylamidopropyl-hydroxysultaïnes, les produits de condensation d'acides gras et d'hydrolysats de protéines, ces composés pouvant être utilisés seuls ou en mélange. Les gammes des Miranol et Mirataines peuvent notamment convenir à la réalisation de la présente invention.

Les tensioactifs non ioniques ou anioniques convenant à l'invention sont par exemple le laurylsulfate de sodium et les alkylglucosides.

Lorsque les nanofibrilles de cellulose mises en oeuvre selon l'invention sont associées à un ou plusieurs composés polyhydroxylés et le cas échéant co-additif(s) précités, la proportion en composé(s) polyhydroxylé(s) et co-additifs(s) est inférieure ou égale à 30 % en poids par rapport au poids en nanofibrilles, composé(s) polyhydroxylé(s) et co-additif(s).

Selon un mode de réalisation particulier de l'invention, ces compositions solides redispersables de nanofibrilles de cellulose essentiellement amorphes, comprennent de la cellulose carboxylée à haut degré de substitution et en tant que co-additif au moins un composé choisi parmi les tensioactifs.

Les formulations buccodentaires selon l'invention incorporant les nanofibrilles de cellulose sous une forme solide redispersable, c'est-à-dire en mélange avec au moins un additif et le cas échéant un co-additif, comprennent également les autres composants classiques de l'application visée.

C'est ainsi que les nanofibrilies de cellulose essentiellement amorphes, chargées de préférence en acides, et associées à un composé polyhydroxylé et éventuellement à un co-additif, sont présentes dans la formulation revendiquée en mélange avec au moins un agent humectant, des abrasifs minéraux insolubles, d'autres agents épaississants et divers composants conventionnels.

Parmi les agents humectants pouvant être mis en oeuvre, on peut citer par exemple le glycérol, le sorbiol, les polyéthylène glycols, les polypropylène glycols, le lactilol, le xylitol, ou leurs mélanges. Cet agent humectant peut représenter de l'ordre de 2 à 85 %, de préférence de l'ordre de 3 à 55 % en poids de ladite formulation buccodentaire, exprimé en matières sèches.

Comme agents abrasifs, on peut mentionner en particulier la silice amorphe, le carbonate de calcium, l'alumine hydratée, la bentonite, le silicate d'aluminium, le silicate de zirconium, les métaphosphates et phosphates de sodium, de potassium, de calcium et de magnésium. Ces poudres abrasives peuvent constituer de l'ordre de 5 à 50 % en poids de la formulation buccodentaire.

Parmi les agents épaississants annexes, on peut mentionner tout particulièrement les silices épaississantes en quantité de l'ordre de 1 à 15 % en poids de ladite composition, les carraghénanes, les dérivés de cellulose, des gommes comme la gomme xanthane, la gomme guar, les alginates, en quantité pouvant aller jusqu'à 5 % en poids de ladite formulation.

Enfin, la formulation buccodentaire peut en outre comporter des agents tensioactifs lavants tels que ceux définis précédemment à titre de co-additifs, des agents détergents, des colorants, des antibactériens, des dérivés fluorés, des opacifiants, des arômes, des édulcorants, des agents antitartre, antiplaque, des agents de blanchiment, du bicarbonate de sodium, des antiseptiques, des enzymes, des extraits naturels (camomille, thym ...)...

La formulation buccodentaire comporte généralement 5 à 60 % en eau .

Il est clair que le choix de ces composés dits conventionnels et l'appréciation de leurs quantités respectives sont directement liés au type de formulation envisagée, à savoir pâte, gel, ou eau de brossage dentaire. Dans le cas particulier des pâtes dentaires, la concentration en charges minérales sera par exemple privilégiée. Ces ajustements relèvent en fait d'opérations de routine pour l'homme du métier.

Au sens de la présente invention, on entend couvrir par formulation buccodentaire, toute formulation de type pâte, liquide ou gel dentaire ainsi que les solutions ou sprays de brossage.

Les nanofibrilles entrant dans les formulations selon l'invention peuvent être obtenues à partir de différents procédés, déjà décrits dans la littérature.

En particulier on pourra se référer au procédé décrit dans la demande de brevet européen EP-A-726 356.

Le traitement y est effectué sur de la pulpe de végétaux à parois primaires à savoir de la pulpe humide, déshydratée, conservée par ensilage ou partiellement dépectinée, comme par exemple de la pulpe de betterave, après que celle-ci a subi une étape d'extraction préalable du saccharose, selon les méthodes connues de la technique. Plus précisément, il comprend une première extraction acide ou basique, à l'issue de laquelle on récupère un premier résidu solide, suivie éventuellement d'une seconde extraction, effectuée dans des conditions alcalines, du premier résidu solide, la récupération d'un second résidu solide, le lavage puis le blanchiment des deux résidus de matériau cellulosique réunis, la dilution du troisième résidu solide obtenu à l'issue de l'étape de blanchiment, puis la dilution de la suspension résultante, de manière à obtenir un taux de matières sèches compris entre 2 et 10 % en poids, et enfin une étape d'homogénéisation, comprenant, au moins un cycle de la suspension diluée.

Plus précisément, l'étape d'homogénéisation, correspond à une opération de mixage, broyage ou toute opération de cisaillement mécanique élevé, suivie d'un ou plusieurs passages de la suspension de cellules à travers un orifice de petit diamètre, soumettant la suspension à une chute de pression d'au moins 20 mPa et à une action de cisaillement à vitesse élevée suivie d'un impact de décélération à vitesse élevée. L'homogénéisation de la suspension cellulosique est obtenue par un nombre de passages pouvant varier entre 1 et 20, de préférence ente 2 et 5, jusqu'à l'obtention d'une suspension stable.

Le procédé qui vient d'être décrit permet d'obtenir des nanofibrilles qui conservent à leur surface des acides carboxyliques et/ou des polysaccharides.

Les nanofibrilles de cellulose sont mises en oeuvre dans les compositions revendiquées sous une forme additivée avec un composé polyhydroxylé de type, par exemple, cellulose carboxylée. Ce dernier peut être introduit dans le protocole de préparation décrit ci-dessus, soit avant la mise en oeuvre de l'étape d'homogénéisation soit après qu'un cycle d'homogénéisation a été opéré.

Il est à noter que cette variante de procédé est décrite dans la demande internationale WO 98/02487.

Le procédé de préparation des nanofibrilles de cellulose additivées en composé(s) polyhydroxylé(s) consiste, dans une première étape, à ajouter à la suspension de nanofibrilles, éventuellement ayant subi au moins un cycle d'homogénéisation, au moins une partie du composé polyhydroxylé considéré et éventuellement du ou des co-additifs. Puis, dans une seconde étape, on met en oeuvre une étape de séchage de la suspension ainsi additivée.

L'exemple 1 présenté ci-après illustre un mode particulier de préparation de nanofibrilles de cellulose additivées en cellulose carboxyméthylée.

En fait l'addition d'au moins une partie du composé polyhydroxylé et éventuellement du ou des co-additifs peut être effectuée selon trois variantes :
- soit à l'issue de l'étape d'homogénéisation et selon un mode privilégié, après que cette dernière a subi au moins une étape de concentration,
- soit à la suspension à l'issue de l'étape d'homogénéisation, avant que cette dernière ait subi au moins une étape de concentration, ou encore
- avant ou pendant l'étape d'homogénéisation, la pulpe ayant alors subi au moins un cycle de l'étape d'homogénéisation.

L'étape ou les étapes de concentration peuvent être effectuées par tout moyen conventionnel jusqu'à l'obtention d'un extrait sec environ 35 % en poids. Plus particulièrement, l'extrait sec est compris entre 5 et 25 % en poids.

Préalablement à l'étape de séchage proprement dite, il peut être avantageux d'effectuer une mise en forme, à savoir par extrusion ou granulation, de la suspension qui a été concentrée. La température de l'étape de séchage est bien entendu retenue de manière à limiter toute dégradation des acides carboxyliques, des polysaccharides acides, des hémicelluloses et/ou des composés polyhydroxylés et co-additifs. Elle est plus particulièrement comprise entre 30 et 80°C, de préférence entre 30 et 60°C.

L'étape de séchage, réalisée par des moyens conventionnels, est effectuée de manière à maintenir au minimum 3 % en poids d'eau par rapport au poids du solide obtenu. Plus particulièrement, le poids d'eau maintenu est compris entre 10 et 30 % en poids. Une telle mise en oeuvre permet de ne pas dépasser le seuil au-delà duquel la redispersion des nanofibrilles ne peut plus être complète.

Avantageusement, la suspension de nanofibrilles de cellulose obtenue par redispersion dans l'eau du mélange, obtenu selon le protocole décrit précédemment, présente un niveau de viscosité correspondant à au moins 50 % pour un taux de cisaillement d'au moins 1 s⁻¹, du niveau de viscosité d'une suspension de nanofibrilles de cellulose n'ayant pas subi d'étape de séchage et ne comprenant pas de composé polyhydroxylé ni de co-additifs.

La présente invention a aussi pour objet l'utilisation des nanofibrilles de cellulose essentiellement amorphes possédant un taux de cristallinité inférieur ou égal à 50 %, telles que définies précédemment, associées à au moins un composé organique polyhydroxylé (poly OH) et éventuellement à un co-additif, tels que définis précédemment, comme agent épaississant et/ou comme agent renforçateur d'arômes dans des formulations buccodentaires.

Elle vise également un procédé pour épaissir une formulation buccodentaire caractérisé en ce qu'il comprend l'addition à ladite formulation de nanofibrilles de cellulose essentiellement amorphes possédant un taux de cristallinité inférieur ou égal à 50 % associées à au moins un composé polyhydroxylé et éventuellement à un co-additif, tels que définis précédemment.

Les exemples soumis ci-après sont présentés à titre illustratif.

### EXEMPLE 1

### Préparation d'un mélange à base de nanofibrilles de cellulose et de carboxyméthylcellulose de haut degré de substitution

Une carboxyméthylcellulose, de haut degré de substitution égal à 1,2 (CMC BLANOSE 12M8P d'AQUALON) est mise en solution dans de l'eau distillée.

La solution est ensuite ajoutée à la dispersion-mère de nanofibrilles à 2,3 % en nanofibrilles de cellulose et préhomogénéisée à l'Ultra-Turrax à 14 000 tr/mn (1 mn pour 100 g de dispersion). L'ensemble est agité à la pâle défloculeuse à 1 000 tr/mn pendant 30 mn.

La quantité de carboxyméthylcellulose ajoutée est de 15 % en poids, par rapport au poids de nanofibrilles de cellulose et de carboxyméthylcellulose.

Le mélange est ensuite versé dans des coupelles puis séché soit dans une étuve ventilée à 40°C, jusqu'à un extrait sec de 92 %, contrôlé par dosage d'eau par la méthode KARL-FISCHER.

Le mélange séché est ensuite broyé, puis tamisé sur un tamis de 500 µm.

La poudre obtenue est redispersée à raison de 0,3 % en poids de nanofibrilles de cellulose dans de l'eau distillée. L'agitation s'effectue à la pale défloculeuse à 1 000 tr/mn pendant 5 mn ou 30 mn.

### EXEMPLE 2

### Préparation de la formulation conforme à l'invention et de formulations témoins

On prépare la formulation selon l'invention incorporant :
- un mélange solide de nanofibrilles de cellulose/carboxyméthylcellulose préparé selon l'exemple 1, à raison de 0,2 % de nanofibrilles, exprimé en poids sec : formulation NFC/CMC.

Elle est comparée à deux formulations témoins :
- une formulation à base de gomme xanthane, incorporée à une concentration en matière active de 0,8 % et
- une formulation incorporant simplement la carboxyméthylcellulose à une concentration en matière active de 1,2 %.

Le tableau 1 présenté ci-après répertorie la nature des autres composants de chacune des formulations testées ainsi que leurs quantités respectives.

**TABLEAU 1**

| INGREDIENTS | Témoin Gomme Xanthane | Témoin CMC | NFC/ CMC |
|---|---|---|---|
| Rhodicare S® | 0,8 | | |
| Blanose 12M8P® | | 1,2 | |
| Dispersion de NFC à 2,3% | | | |
| Mélange NFC/CMC | | | 0,2 |
| Sorbitol à 70 % | 40,9 | 40,9 | 67,14 |
| (Neosorb 70/70® ) | | | |
| Monofluorophosphate de | 0,76 | 0,76 | 0,76 |
| sodium (Na MFP) | | | |
| Saccharinate de sodium | 0,2 | 0,2 | 0,2 |
| Benzoate de sodium | 0,2 | 0,2 | 0,2 |
| Tixosil 73 | 10,0 | 10,0 | 10,0 |
| Tixosil 43 | 10,0 | 10,0 | 10,0 |
| TiO₂ | 1,0 | 1,0 | 1,0 |
| Laurylsulfate de sodium | | | |
| (30%) | 4,2 | 4,2 | 4,2 |
| Arôme menthe verte | 1,0 | 1,0 | 1,0 |
| eau | qs 100 | qs 100 | 6,3 |

### 1) Préparation des formulations témoins

On dissout des sels de sodium en milieu aqueux et la solution ainsi obtenue est mélangée avec du Neosorb® 70/70. Il est ensuite dispersé au sein du mélange, à l'aide d'un homogénéiseur de type Rayneri, soit de la gomme xanthane (Rhodicare S® ) ou de la carboxyméthylcellulose (Blanose 12M8P® ) pendant une minute à 600 tours/minute, puis on procède l'hydratation (pendant 15 minutes à 2 000 tours/minutes). L'ensemble du mélange est ensuite transféré dans un malaxeur de type Guédu® et laissé reposé pendant 30 minutes. On ajoute alors sous agitation les silices et l'oxyde de titane. Au terme de 10 minutes d'agitation, l'ensemble est refroidi et est porté sous vide. L'ensemble de cette opération agitation-refroidissement est réalisé en une heure. On diminue ensuite la vitesse d'agitation et on procède à l'addition du tensioactif et de l'arôme, sous vide. L'ensemble est mélangé pendant 5 minutes puis le vide est cassé, le malaxeur vidé et la pâte ainsi obtenue conditionnée.

### 2) Préparation de la formulation selon l'invention

On met en oeuvre le mélange NFC-CMC contenant 15 % en CMC de haut degré de substitution préparé selon l'exemple 1. Ce mélange solide, d'extrait sec de 92 %, est dispersé à raison de 0,16 % en poids à l'aide d'un homogénéiseur de type Rayneri® dans un mélange eau-Neosorb® 70/70 à 600 tours/minutes puis on procède à une hydratation pendant 30 minutes à 10 000 tours par minute. L'ensemble est ensuite transféré dans le malaxeur où il ne subit aucune phase de repos. On ajoute ensuite sous agitation les silices, l'oxyde de titane et les sels de sodium. Au terme de 10 minutes, l'ensemble est refroidi et placé sous vide, l'opération étant réalisée en temps total pendant 1 heure.

Pour la suite, on procède conformément au procédé décrit dans le cas de la mise au point des formulations témoins.

### EXEMPLE 3

### Evaluation des propriétés des pâtes dentaires formulées selon l'exemple 1

Cette évaluation est réalisée après vieillissement d'une semaine. Les critères retenus sont :
- l'aspect évalué visuellement ; il est noté de 0 (mauvais aspect) à ++ (bel aspect) et
- la consistance, mesurée à l'aide d'un viscosimètre de type Brookfield RV 8® , muni d'un pied hélipathe, à 5 tours par minute.

Les résultats observés figurent dans le tableau 2 ci-après.

**TABLEAU 2**

| Composition | Aspect | Consistance (Pa.s) |
|---|---|---|
| Témoin Gomme xanthane | ++ | 123 |
| Témoin CMC | ++ | 145 |
| NFC/CMC | ++ | 320 |

De l'examen de ces résultats, il ressort que les nanofibrilles de cellulose sous forme associée à au moins un composé organigue polyhydroxylé donnent des pâtes dentaires ayant des consistances avantageuses et ceci plus particulièrement pour une concentration de l'ordre de 0,15 % en poids.

### EXEMPLE 4

### Evaluation de l'effet renforçateur d'arômes des nanofibrilles de cellulose

Cette évaluation est réalisée par analyse sensorielle effectuée sur 10 personnes en testant une formulation buccodentaire conforme à l'invention, au regard d'une formulation buccodentaire témoin, sans nanofibrilles de cellulose.

Les compositions de chacune des formulations testées sont identifiées ci-après.

La composition NFC/CMC utilisée comprend 70 % en poids de NFC pures, 5 % en poids de CMC (Blanose 7HXF) et 25 % en poids de sorbitol. Cette composition est dénommée NFC/CMC/sorbitol dans la formulation buccodentaire testée ci-après.

| Formulation buccodentaire avec NFC/CMC | |
|---|---|
| Produit | % p/p |
| NFC/CMC/sorbitol | 0,25 |
| Neosorb® 70/70 | 67,09 |
| Monofluorophosphate de sodium | 0,76 |
| Saccharinate de sodium | 0,2 |
| Benzoate de sodium | 0,2 |
| Tixosil 73 | 10,0 |
| Tixosil 43 | 10,0 |
| TiO₂ | 1,0 |
| Laurylsulfate de sodium Sipon LCS 98 (solution à 30 %) | 4,2 |
| Arôme Herbal® de la Société Mane | 1,0 |
| Eau q.s.p. | 100 |

| Formulation buccodentaire avec CMC | |
|---|---|
| Produit | % p/p |
| Blanose 7HXF | 0,4 |
| Neosorb® 70/70 | 66,87 |
| Monofluorophosphate de sodium | 0,76 |
| Saccharinate de sodium | 0,2 |
| Benzoate de sodium | 0,2 |
| Tixosil 73 | 10,0 |
| Tixosil 43 | 10,0 |
| TiO₂ | 1,0 |
| Laurylsulfate de sodium Sipon LCS 98 (solution à 30 %) | 4,2 |
| Arôme Herbal® de la Société Mane | 1,0 |
| Eau q.s.p. | 100 |

La formulation selon l'invention est obtenue par dispersion et hydratation des NFC/CMC dans du Neosorb® 70/70 10 mn à l'Ultra-Turrax à 24 000 trs/mn et à la pale défloculeuse 30 mn à 2 000 trs/mn, par transfert immédiat du mélange dans un malaxeur. On y introduit ensuite les silices, les sels de sodium et l'oxyde de titane sous agitation. L'ensemble est mélangé 1 heure dont 50 mn sous vide et sous refroidissement à 23 °C. Le tensioactif et l'arôme sont ensuite introduits sous agitation et sous vide. L'ensemble est mélangé 5 mn puis l'on vide et l'on récupère le contenu du malaxeur.

La formulation témoin est obtenue par dispersion et hydratation de la CMC dans le Neosorb® 70/70 à la pale défloculeuse 15 mn à 2 000 trs/mn. A l'issue de cette opération, l'ensemble est transféré dans un malaxeur et l'on procède selon le protocole décrit ci-dessus pour obtenir la formulation témoin.

Les critères retenus pour cette évaluation ainsi que les résultats obtenus sont soumis dans le tableau III ci-après. Chaque critère est noté de 0 (mauvais comportement) à +++ (bon comportement).

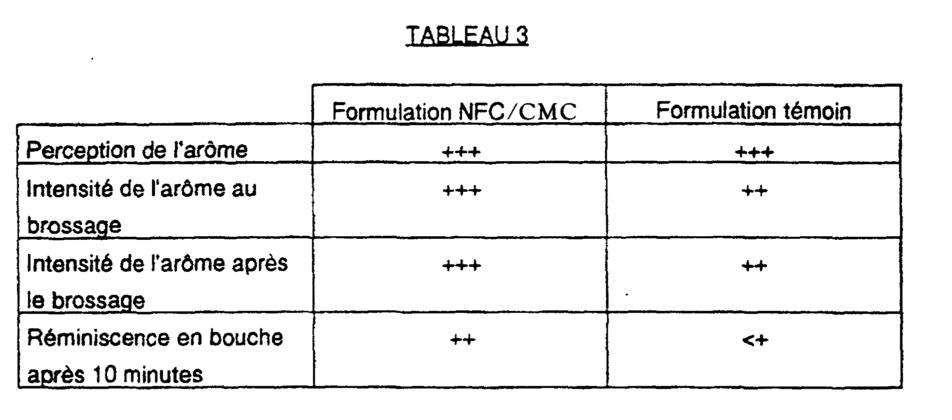

## Revendications

1. Formulation buccodentaire **caractérisée en ce qu'**elle comprend au moins un agent épaississant comprenant des nanofibrilles de cellulose essentiellement amorphes, possédant un taux de cristallinité inférieur ou égal à 50 %, lesdites nanofibrilles de cellulose étant présentes sous une forme associée à au moins un composé organique polyhydroxylé (polyOH) dans un rapport pondéral (polyOH) / [(polyOH) + (NFC)] inférieur ou égal à 30 %.

2. Formulation buccodentaire selon la revendication 1 **caractérisé en ce que** le taux de cristallinité est compris entre 15 et 50 % et de préférence inférieur à 50 %.

3. Formulation buccodentaire selon l'une des revendications 1 ou 2 **caractérisée en ce que** les nanofibrilles de cellulose sont issues de cellules constituées de préférence d'au moins environ 80 % de parois primaires.

4. Formulation buccodentaire selon la revendication 3 **caractérisée en ce que** la quantité de parois primaires est d'au moins 85 % en poids.

5. Formulation buccodentaire selon l'une quelconque des revendications 1 à 4, **caractérisée en ce que** les nanofibrilles de cellulose présentent au moins 80 % de cellules à parois primaires.

6. Formulation buccodentaire selon l'une quelconque des revendications 1 à 5 **caractérisée en ce qu'**elle comprend environ 0,1 à 0,4 % en poids desdites nanofibrilles de cellulose.

7. Formulation buccodentaire selon la revendication 6 **caractérisée en ce qu'**elle comprend environ 0,15 à 0,3 % en poids desdites nanofibrilles de cellulose.

8. Formulation buccodentaire selon l'une quelconque des revendications précédentes **caractérisée en ce que** les nanofibrilles de cellulose sont en outre chargées en surface en acides carboxyliques, en polysaccharides acides, seuls ou en mélange.

9. Formulation buccodentaire selon l'une quelconque des revendications 1 à 8 **caractérisée en ce que** le composé organique polyhydroxylé (polyOH) est choisi parmi les carbohydrates et leurs dérivés et les polyalcools.

10. Formulation buccodentaire selon la revendication 9 **caractérisée en ce que** les carbohydrates sont de préférence choisis parmi les monosaccharides linéaires ou cycliques en C3 à C6, les oligosaccharides, les polysaccharides et leurs dérivés gras comme les sucroesters d'acides gras, les carbohydrates alcools, acides ou éthers.

11. Formulation buccodentaire selon l'une quelconque des revendications 1 à 10 **caractérisée en ce que** le composé organique polyhydroxylé (polyOH) est de préférence choisi parmi la carboxyméthylcellulose, la gomme xanthane, les guars, le sorbitol et leurs mélanges.

12. Formulation buccodentaire selon la revendication 11 **caractérisée en ce que** les nanofibrilles de cellulose y sont associées à de la carboxyméthylcellulose (CMC) de haut degré de substitution, dans un rapport pondéral (CMC) / [(CMC)+(NFC)] inférieur ou égal à 20 %.

13. Formulation buccodentaire selon l'une quelconque des revendications 1 à 12 **caractérisée en ce que** les nanofibrilles de cellulose y sont en outre associées à au moins un co-additif choisi parmi :
- les composés de formule (R1R2N)COA, dans laquelle R1 ou R2, identiques ou différents, représentent l'hydrogène ou un radical alkyle en C1-C10, de préférence en C1-C5, A représente l'hydrogène, un radical alkyle en C1-C10, de préférence en C1-C5, ou encore le groupement R'¹R'²N avec R'1, R'2, identiques ou différents, représentant l'hydrogène ou un radical alkyle en C₁-C₁₀, de préférence en C₁-C₅ et
- les tensioactifs de préférence non ioniques, anioniques et amphotères et leurs mélanges.

14. Formulation buccodentaire selon l'une quelconque des revendications 1 à 13 **caractérisée en ce que** la teneur en composé(s) polyhydroxylé(s) et en co-additif(s) éventuel(s) est inférieure ou égale à 30 % en poids par rapport au poids en nanofibrilles, composé(s) polyhydroxylé(s) et de co-additif(s).

15. Utilisation de nanofibrilles de cellulose essentiellement amorphes, possédant un taux de cristallinité inférieur ou égal à 50 %, lesdites nanofibrilles de cellulose étant présentes sous une forme associée à au moins un composé organique polyhydroxylé (polyOH) dans un rapport pondéral (polyOH) / [(polyOH) + (NFC)] inférieur ou égal à 30 % telles que définies à l'une quelconque des revendications 1 à 12, à titre d'agent épaississant dans les formulations buccodentaires.

16. Utilisation de nanofibrilles selon la revendication 15, dans laquelle les nanofibrilles de cellulose sont, en outre, associées à au moins un co-additif tel que décrit à la revendication 13.

17. Utilisation de nanofibrilles de cellulose essentiellement amorphes, possédant un taux de cristallinité inférieur ou égal à 50 %, lesdites nanofibrilles de cellulose étant présentes sous une forme associée à au moins un composé organique polyhydroxylé (polyOH) dans un rapport pondéral (polyOH) / [(polyOH) + (NFC)] inférieur ou égal à 30 % telles que définies à l'une quelconque des revendications 1 à 12, à titre d'agent renforçateur d'arômes dans les formulations buccodentaires.

18. Utilisation de nanofibrilles selon la revendication 17, dans laquelle les nanofibrilles de cellulose sont, en outre, associées à au moins un co-additif tel que décrit à la revendication 13.

19. Procédé pour épaissir une formulation buccodentaire **caractérisé en ce qu'**il comprend l'addition à ladite formulation, de nanofibrilles de cellulose essentiellement amorphes, possédant un taux de cristallinité inférieur ou égal à 50 %, lesdites nanofibrilles de cellulose étant sous une forme associée à au moins un composé organique polyhydroxylé (polyOH) dans un rapport pondéral (polyOH) / [(polyOH) + (NFC)] inférieur ou égal à 30 % telles que définies à l'une quelconque des revendications 1 à 12.

20. Procédé pour épaissir une formulation buccodentaire selon la revendication 19, **caractérisé en ce qu'**il comprend l'addition d'un un co-additif tel que défini à la revendication 13.

## Claims

1. Dentibuccal formulation, **characterized in that** it includes at least one thickening agent comprising essentially amorphous cellulose nanofibrils, having a degree of crystallinity less than or equal to 50%, said cellulose nanofibrils being present in a form combined with at least one polhydroxylated organic compound (polyOH) in a weight ratio (polyOH) / [(polyOH) + (NFC)] less than or equal to 30%.

2. Dentibuccal formulation according to claim 1, **characterized in that** the degree of crystallinity is between 15 and 50% and preferably less than 50%.

3. Dentibuccal formulation according to one of claims 1 or 2, **characterized in that** the cellulose nanofibrils have come from cells preferably at least about 80% constituted by primary walls.

4. Dentibuccal formulation according to claim 3, **characterized in that** the quantity of primary walls is at least 85% by weight.

5. Dentibuccal formulation according to any one of claims 1 to 4, **characterized in that** the cellulose nanofibrils present at least 80% by weight of cells with primary walls.

6. Dentibuccal formulation according to any one of claims 1 to 5, **characterized in that** it includes about 0.1 to 0.4% by weight of said cellulose nanofibrils.

7. Dentibuccal formulation according to claim 6, **characterized in that** it includes about 0.15 to 0.3% by weight of said cellulose nanofibrils.

8. Dentibuccal formulation according to any one of the previous claims, **characterized in that** the cellulose nanofibrils are also surface-loaded with carboxylic acids, acid polysaccharides, alone or mixed.

9. Dentibuccal formulation according to any one of claims 1 to 8, **characterized in that** the polyhydroxylated organic compound (polyOH) is chosen from among carbohydrates and their derivatives and polyalcohols.

10. Dentibuccal formulation according to claim 9, **characterized in that** the carbohydrates are preferably chosen from among linear or cyclic C3 to C6 monosaccharides, oligosaccharides, polysaccharides and their fatty derivatives such as sucroesters of fatty acids, alcohol carbohydrates, acids or ethers.

11. Dentibuccal formulation according to any one of claims 1 to 10, **characterized in that** the polyhydroxylated organic compound (polyOH) is preferably chosen from among carboxymethylcellulose, xanthan gum, guars, sorbitol and their mixtures.

12. Dentibuccal formulation according to claim 11, **characterized in that** the cellulose nanofibrils are combined there with carboxymethylcellulose (CMC) with a high degree of substitution, in a weight ratio (CMC) / [(CMC) + (NFC)] less than or equal to 20%.

13. Dentibuccal formulation according to any one of claims 1 to 12, **characterized in that** the cellulose nanofibrils are also combined with at least one co-additive chosen from among:
- compounds of formula (R1R2N)COA, in which R1 or R2, identical or different, represent hydrogen or a C1-C10, preferably C1-C5, alkyl radical, A represents hydrogen, a C1-C10, preferably C1-C5, alkyl radical, or else the grouping R'1R'2N, with R'1, R'2, identical or different, representing hydrogen or a C1-C10, preferably C1-C5, alkyl radical, and
- surfactants, preferably ionic, anionic and amphoteric, and their mixtures.

14. Dentibuccal formulation according to any one of claims 1 to 13, **characterized in that** the level of polyhydroxylated compound(s) and any co-additive(s) is less than or equal to 30% by weight relative to the weight in nanofibrils, polyhydroxylated compound(s) and co-additive(s).

15. Use of essentially amorphous cellulose nanofibrils, having a degree of crystallinity less than or equal to 50%, said cellulose nanofibrils being present in a form combined with at least one polyhydroxylated organic compound (polyOH) in a weight ratio (polyOH) / [(polyOH) + (NFC)] less than or equal to 30% as defined in any one of claims 1 to 12, as thickening agent in dentibuccal formulations.

16. Use of nanofibrils according to claim 15, in which the cellulose nanofibrils are also combined with at least one co-additive as described in claim 13.

17. Use of essentially amorphous cellulose nanofibrils, having a degree of crystallinity less than or equal to 50%, said cellulose nanofibrils being present in a form combined with at least one polhydroxylated organic compound (polyOH) in a weight ratio (polyOH) / [(polyOH) + (NFC)] less than or equal to 30% as defined in any one of claims 1 to 12, as aroma-reinforcing agent in dentibuccal formulations.

18. Use of nanofibrils according to claim 17, in which the cellulose nanofibrils are also combined with at least one co-additive as described in claim 13.

19. Process for thickening a dentibuccal formulation, **characterized in that** it comprises the addition to the said formulation of essentially amorphous cellulose nanofibrils, having a degree of crystallinity less than or equal to 50%, said cellulose nanofibrils being present in a form combined with at least one polhydroxylated organic compound (polyOH) in a weight ratio (polyOH) / [(polyOH) + (NFC)] less than or equal to 30% as defined in any one of claims 1 to 12.

20. Process for thickening a dentibuccal formulation according to claim 19, **characterized in that** it comprises the addition of a co-additive as defined in claim 13.

## Patentansprüche

1. Buccodentale Formulierung, welche **dadurch gekennzeichnet ist, dass** diese wenigstens ein Verdickungsmittel umfasst, welches Nanofibrillen aus Cellulose, die im wesentlichen amorph sind, umfasst, welche einen Kristallinitätsgrad besitzen, der kleiner oder gleich 50 % ist, wobei die Nanofibrillen aus Cellulose in einer Form vorliegen, die mit mindestens einer organischen polyhydroxylierten Verbindung (polyOH) in einem Gewichtsverhältnis (polyOH) / [(polyOH) + (NFC)], das kleiner oder gleich 30 % ist, assoziiert ist.

2. Buccodentale Formulierung nach Anspruch 1, **dadurch gekennzeichnet, dass** der Kristallinitätsgrad zwischen 15 und 50 % und vorzugsweise unter 50 % liegt.

3. Buccodentale Formulierung nach einem der Ansprüche 1 oder 2, **dadurch gekennzeichnet, dass** die Nanofibrillen aus Cellulose aus Zellen stammen, die vorzugsweise aus wenigstens ca. 80 % Primärwänden bestehen.

4. Buccodentale Formulierung nach Anspruch 3, **dadurch gekennzeichnet, dass** die Menge an Primärwänden wenigstens 85 Gew.-% beträgt.

5. Buccodentale Formulierung nach einem der Ansprüche 1 bis 4, **dadurch gekennzeichnet, dass** die Nanofibrillen aus Cellulose wenigstens 80 % der Zellen mit Primärwänden ausmachen.

6. Buccodentale Formulierung nach einem der Ansprüche 1 bis 5, **dadurch gekennzeichnet, dass** diese ca. 0,1 bis 0,4 Gew.-% der Nanofibrillen aus Cellulose umfasst.

7. Buccodentale Formulierung nach Anspruch 6, **dadurch gekennzeichnet, dass** diese ca. 0,15 bis 0,3 Gew.-% der Nanofibrillen aus Cellulose umfasst.

8. Buccodentale Formulierung nach einem der vorhergehenden Ansprüche, **dadurch gekennzeichnet, dass** die Nanofibrillen aus Cellulose außerdem an der Oberfläche durch Carbonsäuren, durch saure Polysaccharide, allein oder in einer Mischung, geladen sind.

9. Buccodentale Formulierung nach einem der Ansprüche 1 bis 8, **dadurch gekennzeichnet, dass** die organische polyhydroxylierte Verbindung (polyOH) aus den Kohlenhydraten und deren Derivaten und den Polyalkoholen ausgewählt ist.

10. Buccodentale Formulierung nach Anspruch 9, **dadurch gekennzeichnet, dass** die Kohlenhydrate vorzugsweise aus den linearen oder cyclischen Monosacchariden mit C₃ bis C₆, den Oligosacchariden, den Polysacchariden und deren Fettderivaten wie den Zuckerestern der Fettsäuren, den Kohlenhydratalkoholen, -säuren oder -ethern ausgewählt sind.

11. Buccodentale Formulierung nach einem der Ansprüche 1 bis 10, **dadurch gekennzeichnet, dass** die organische polyhydroxylierte Verbindung (polyOH) vorzugsweise aus der Carboxymethylcellulose, dem Xanthangummi, den Guargummis, dem Sorbitol und deren Mischungen ausgewählt ist.

12. Buccodentale Formulierung nach Anspruch 11, **dadurch gekennzeichnet, dass** die Nanofibrillen aus Cellulose darin mit Carboxymethylcellulose (CMC) mit einem hohen Substitutionsgrad, in einem Gewichtsverhältnis (CMC) / [(CMC) + (NFC)], das kleiner oder gleich 20 % ist, assoziiert sind.

13. Buccodentale Formulierung nach einem der Ansprüche 1 bis 12, **dadurch gekennzeichnet, dass** die Nanofibrillen aus Cellulose darin außerdem mit wenigstens einem Zusatz assoziiert sind, der ausgewählt ist aus:
- den Verbindungen der Formel (R¹R²N)COA, in welcher R¹ oder R², die gleich oder verschieden sind, für Wasserstoff oder eine C₁-C₁₀-, vorzugsweise eine C₁-C₅-Alkylgruppe stehen, A für Wasserstoff, eine C₁-C₁₀-, vorzugsweise eine C₁-C₅-Alkylgruppe steht, oder auch der Gruppierung R'¹R'²N, wobei R'¹, R'², die gleich oder verschieden sind, für Wasserstoff oder eine C₁-C₁₀-, vorzugsweise eine C₁-C₅-Alkylgruppe stehen, und
- den Tensiden, die vorzugsweise nichtionisch, anionisch und amphoter sind, sowie deren Mischungen.

14. Buccodentale Formulierung nach einem der Ansprüche 1 bis 13, **dadurch gekennzeichnet, dass** der Gehalt der polyhydroxylierten Verbindung(en) und des fakultativen Zusatzes (der fakultativen Zusätze) kleiner oder gleich 30 Gew.-%, bezogen auf das Gewicht der Nanofibrillen, der polyhydroxylierten Verbindung(en) und des Zusatzes (der Zusätze), beträgt.

15. Verwendung von Nanofibrillen aus Cellulose, die im wesentlichen amorph sind, wobei diese einen Kristallinitätsgrad von kleiner oder gleich 50 % besitzen, wobei die Nanofibrillen aus Cellulose in einer Form vorliegen, die mit wenigstens einer organischen polyhydroxylierten Verbindung (polyOH) in einem Gewichtsverhältnis (polyOH) / [(polyOH) + (NFC)], das kleiner oder gleich 30 % ist, assoziiert ist, so wie sie in einem der Ansprüche 1 bis 12 definiert sind, in der Eigenschaft als Verdickungsmittel in den buccodentalen Formulierungen.

16. Verwendung von Nanofibrillen nach Anspruch 15, bei welcher die Nanofibrillen aus Cellulose außerdem mit wenigstens einem Zusatz assoziiert sind, wie er in Anspruch 13 beschrieben wird.

17. Verwendung von Nanofibrillen aus Cellulose, die im wesentlichen amorph sind, wobei diese einen Kristallinitätsgrad von kleiner oder gleich 50 % besitzen, wobei die Nanofibrillen aus Cellulose in einer Form vorliegen, die mit wenigstens einer organischen polyhydroxylierten Verbindung (polyOH) in einem Gewichtsverhältnis (polyOH) / [(polyOH) + (NFC)], das kleiner oder gleich 30 % ist, assoziiert ist, so wie sie in einem der Ansprüche 1 bis 12 definiert sind, in der Eigenschaft als Geschmacksverstärkungsmittel in den buccodentalen Formulierungen.

18. Verwendung von Nanofibrillen nach Anspruch 17, bei welcher die Nanofibrillen aus Cellulose außerdem mit wenigstens einem Zusatz, wie er in Anspruch 13 beschrieben wird, assoziiert sind.

19. Verfahren zum Verdicken einer buccodentalen Formulierung, **dadurch gekennzeichnet, dass** dieses die Zugabe zu der Formulierung von Nanofibrillen aus Cellulose, die im wesentlichen amorph sind, wobei diese einen Kristallinitätsgrad von kleiner oder gleich 50 % besitzen, umfasst, wobei die Nanofibrillen aus Cellulose in einer Form vorliegen, die mit wenigstens einer organischen polyhydroxylierten Verbindung (polyOH) in einem Gewichtsverhältnis (polyOH) / [(polyOH) + (NFC)], das kleiner oder gleich 30 % ist, assoziiert ist, so wie sie in einem der Ansprüche 1 bis 12 definiert sind.

20. Verfahren zum Verdicken einer buccodentalen Formulierung nach Anspruch 19, **dadurch gekennzeichnet, dass** es die Zugabe eines Zusatzes, wie er in Anspruch 13 definiert ist, umfasst.
